(19) Europäisches Patentamt
European Patent Office
Office européen des brevets

(11) EP 4 289 931 A1

(12) EUROPEAN PATENT APPLICATION
published in accordance with Art. 153(4) EPC

(43) Date of publication:
**13.12.2023 Bulletin 2023/50**

(21) Application number: **22749743.5**

(22) Date of filing: **02.02.2022**

(51) International Patent Classification (IPC):
***C12M 3/00*** (2006.01) ***C07K 5/00*** (2006.01)
***C07K 7/06*** (2006.01) ***C12M 1/00*** (2006.01)
***C12N 5/02*** (2006.01) ***C12N 5/071*** (2010.01)

(52) Cooperative Patent Classification (CPC):
**C07K 5/00; C07K 7/06; C12M 1/00; C12M 3/00; C12N 5/0006; C12N 5/06**

(86) International application number:
**PCT/JP2022/004063**

(87) International publication number:
**WO 2022/168872 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2021 JP 2021015724**
**31.08.2021 JP 2021141461**

(71) Applicant: **SEKISUI CHEMICAL CO., LTD.**
**Osaka-shi**
**Osaka**
**530-8565 (JP)**

(72) Inventors:
- **TAKAKURA, Kenta**
**Mishima-gun, Osaka 618-0021 (JP)**
- **HANEDA, Satoshi**
**Mishima-gun, Osaka 618-0021 (JP)**
- **NAKAMURA, Yuuta**
**Mishima-gun, Osaka 618-0021 (JP)**

(74) Representative: **Ter Meer Steinmeister & Partner Patentanwälte mbB**
**Nymphenburger Straße 4**
**80335 München (DE)**

Remarks:
The complete document including Reference Table(s) and the Sequence Listing(s) can be downloaded from the EPO website

# (54) MICROCARRIER FOR CELL CULTURE AND CELL CULTURE METHOD

(57) Provided is a microcarrier for cell culture capable of suppressing breakage of the microcarrier in a cell culture process and improving cell culture efficiency. The microcarrier for cell culture according to the present invention includes a base particle and a coating layer coating an outer surface of the base particle, and has a strength at break of 1000 mN or more.

[FIG. 1.]

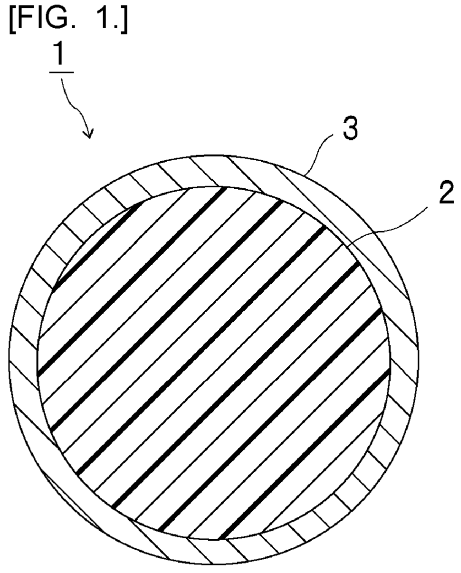


EP 4 289 931 A1

## Description

### TECHNICAL FIELD

The present invention relates to a microcarrier for cell culture. The present invention also relates to a cell culture method using the microcarrier for cell culture.

### BACKGROUND ART

In research and development in academic fields, drug discovery fields, regenerative medicine fields, and other fields, animal cells such as human cells, mouse cells, rat cells, pig cells, cow cells, and monkey cells are used. As a cell culture method, a method using a microcarrier is known.

Conventionally, as the microcarrier, a microcarrier in which a base particle is coated with an extracellular matrix (ECM) is widely used. For example, Patent Document 1 below describes a microcarrier including a polystyrene particle and vitronectin disposed on the outer surface of the polystyrene particle.

A microcarrier in which a base particle is coated with a synthetic resin is also known. For example, Patent Document 2 below describes a microcarrier including a polystyrene particle and a synthetic resin layer disposed on the outer surface of the polystyrene particle. The synthetic resin layer contains a synthetic resin to which a peptide is bonded.

### Related Art Document

### Patent Document

Patent Document 1: WO 2011/017167 A1
Patent Document 2: WO 2011/017050 A1

### SUMMARY OF THE INVENTION

### PROBLEMS TO BE SOLVED BY THE INVENTION

In conventional microcarriers as described in Patent Documents 1 and 2, resin particles having a small degree of crosslinking, such as polystyrene particles, are used as base particles. Thus, in the conventional microcarrier, the microcarrier may be broken in a cell culture process. For example, in the conventional microcarrier, the microcarriers may collide with each other at the time of cell culturing, or impact may be applied to the microcarrier at the time of replacing a culture medium, so that the microcarrier may be chipped or split. Such microcarrier breakage is particularly likely to occur in the process of culturing cells on a scale of several hundred liters or more using a massive culture apparatus.

When the microcarrier is broken, it is difficult to separate fragments of the microcarrier from cells, and therefore, for example, the fragments may be mixed in a cell preparation.

In addition, cell culture efficiency is preferably high.

An object of the present invention is to provide a microcarrier for cell culture capable of suppressing breakage of the microcarrier in a cell culture process and improving cell culture efficiency. Another object of the present invention is to provide a cell culture method using the microcarrier for cell culture.

### MEANS FOR SOLVING THE PROBLEMS

According to a broad aspect of the present invention, there is provided a microcarrier for cell culture (hereinafter may be abbreviated as a microcarrier) including a base particle and a coating layer coating an outer surface of the base particle, and having a strength at break of 1000 mN or more.

In a specific aspect of the microcarrier according to the present invention, a compression displacement curve obtained when a compression test is performed has no inflection point at a load of 700 mN or less.

In a specific aspect of the microcarrier according to the present invention, a water absorption rate is 10% by weight or less.

In a specific aspect of the microcarrier according to the present invention, the coating layer contains a synthetic resin.

In a specific aspect of the microcarrier according to the present invention, the synthetic resin has a polyvinyl

alcohol derivative skeleton or a poly(meth)acrylic acid ester skeleton.

In a specific aspect of the microcarrier according to the present invention, the coating layer contains a peptide moiety.

In a specific aspect of the microcarrier according to the present invention, the base particle is a resin particle.

In a specific aspect of the microcarrier according to the present invention, the base particle contains a polymer of a monomer having an ethylenically unsaturated group.

In a specific aspect of the microcarrier according to the present invention, the polymer of the monomer having the ethylenically unsaturated group is an acrylic resin, a divinylbenzene polymer, or a divinylbenzene copolymer.

In a specific aspect of the microcarrier according to the present invention, a specific gravity is 1.0 g/cm$^3$ or more and 2.0 g/cm$^3$ or less.

In a specific aspect of the microcarrier according to the present invention, an average particle diameter is 100 um or more and 1500 um or less.

In a specific aspect of the microcarrier according to the present invention, a CV value of a particle diameter is 10% or less.

According to a broad aspect of the present invention, there is provided a cell culture method including a step of adhering a cell to the microcarrier for cell culture described above.

## EFFECT OF THE INVENTION

A microcarrier for cell culture according to the present invention includes a base particle and a coating layer coating an outer surface of the base particle, and has a strength at break of 1000 mN or more. Since the microcarrier for cell culture according to the present invention is provided with the above configuration, it is possible to suppress breakage of the microcarrier in a cell culture process and improve cell culture efficiency.

## BRIEF DESCRIPTION OF DRAWINGS

[Fig. 1] Fig. 1 is a cross-sectional view schematically showing a microcarrier for cell culture according to one embodiment of the present invention.

[Fig. 2] Fig. 2 is a photograph of a broken microcarrier observed in Comparative Example 1.

## MODE FOR CARRYING OUT THE INVENTION

Hereinafter, the details of the present invention will be described.

(Microcarrier for cell culture)

A microcarrier for cell culture (hereinafter may be abbreviated as "microcarrier") according to the present invention includes a base particle and a coating layer coating an outer surface of the base particle, and has a strength at break of 1000 mN or more.

Since the microcarrier according to the present invention is provided with the above configuration, it is possible to suppress breakage of the microcarrier in a cell culture process and improve cell culture efficiency.

A conventional microcarrier has relatively low strength. Thus, in the conventional microcarrier, the microcarriers may collide with each other at the time of cell culturing, or impact may be applied to the microcarrier at the time of replacing a culture medium, so that the microcarrier may be chipped or split. Since such breakage of the microcarrier is particularly likely to occur in the process of culturing cells using a massive culture apparatus, it may be difficult to use the conventional microcarrier in cell culture on a scale of several hundred liters.

On the other hand, the strength of the microcarrier according to the present invention is relatively high. Thus, in the microcarrier according to the present invention, even when the microcarriers may collide with each other at the time of cell culturing, or impact may be applied to the microcarrier at the time of replacing a culture medium, the microcarrier may be less likely to be chipped or split. Therefore, in the microcarrier according to the present invention, for example, in a cell preparation, a risk of mixing fragments of the microcarrier can be suppressed to a low level.

In addition, in the microcarrier according to the present invention, the cell culture efficiency can be enhanced.

The microcarrier according to the present invention can be suitably used from cell culture on a scale of several tens of milliliters to cell culture on a scale of several hundreds of liters or more.

In addition, in the microcarrier according to the present invention, since it is not necessary to use a natural polymer material such as an extracellular matrix (ECM) as a material, the microcarrier is inexpensive, has little variation

between lots, and is excellent in safety.

From the viewpoint of suppressing breakage of the microcarrier in the cell culture process, the microcarrier has a strength at break of 1000 mN or more. That is, the microcarrier does not have a strength at break of less than 1000 mN.

The strength at break of the microcarrier is preferably 1100 mN or more, more preferably 1200 mN or more, and still more preferably 1500 mN or more. When the strength at break is the above lower limit or more, breakage of the microcarrier in the cell culture process can be more effectively suppressed. The upper limit of the strength at break of the microcarrier is not particularly limited. The microcarrier may have a strength at break of 10000 mN or less.

The strength at break of the microcarrier is a strength at break when the microcarrier is compressed. The strength at break of the microcarrier is a strength at break when the following compression test is performed. The strength at break of the microcarrier can be measured as follows.

A microcarrier compression test is performed at a smooth indenter end face of a cylinder (diameter of 500 μm, made of diamond) using a micro strength evaluation tester under the conditions of loading at 25°C and a maximum test load of 2000 mN over 0.3 N/sec. The load when the microcarrier breaks is defined as the strength at break of the microcarrier. As the micro strength evaluation tester, for example, "Microautograph MST-I" manufactured by Shimadzu Corporation is used.

The strength at break of the microcarrier can be increased, for example, by using a resin having a high degree of crosslinking as a material of the base particle, using a base particle containing a filler, or using a resin having a flexible molecular structure as a material of the base particle.

In the microcarrier, a compression displacement curve obtained when the compression test is performed preferably has no inflection point at a load of 700 mN or less. In this case, breakage of the microcarrier in the cell culture process can be more effectively suppressed.

The conditions of the compression test for obtaining the compression displacement curve are the same as the conditions of the compression test for obtaining the strength at break. The compression displacement curve can be obtained as follows.

In the microcarrier compression test, a load value (mN) and a compressive displacement (μm) are measured, and the compression displacement curve indicating a relationship between the compressive displacement (x-axis) and the load value (y-axis) is created. A point at which a slope of a tangent of the obtained compression displacement curve changes from an increase to a decrease is defined as the inflection point. It is confirmed whether the obtained compression displacement curve has the inflection point at a load of 700 mN or less.

When the microcarrier is compressed, the slope of the tangent of the compression displacement curve tends to increase; however, when microcarrier is cracked, the slope of the tangent of the compression displacement curve is likely to turn to decline. Therefore, when the obtained compression displacement curve has no inflection point at a load of 700 mN or less, mixing of fragments generated from the microcarrier can be more effectively suppressed.

A water absorption rate of the microcarrier is preferably 10% by weight or less, more preferably 5% by weight or less, and still more preferably 1% by weight or less. When the water absorption rate is the above upper limit or less, a state of a surface of the microcarrier is less likely to change at the time of adhesion of cells, and therefore, it is possible to reduce a variation in an initial fixing rate after cell seeding. When the water absorption rate is the above upper limit or less, cells are less likely to be detached from the microcarrier in the culture medium. The lower limit of the water absorption rate of the microcarrier is not particularly limited. The microcarrier may have a water absorption rate of 0% by weight or more, or 0.001% by weight or more.

The water absorption rate of the microcarrier can be measured as follows.

A microcarrier dried in an oven at 100°C for 8 hours is provided. The microcarrier (100.0 mg) is left to stand for 24 hours in an environment of a temperature of 37°C and a relative humidity of 95% RH. A weight of the microcarrier after being left to stand is measured. The water absorption rate of the microcarrier is calculated by the following formula.

$$\text{Water absorption rate (\% by weight)} = (W_2 - W_1)/W_1 \times 100$$

$W_1$: Weight of microcarrier before being left to stand (mg)
$W_2$: Weight of microcarrier after being left to stand (mg)

Examples of a method of reducing the water absorption rate of the microcarrier include preparing a coating layer using a material having high hydrophobicity.

A specific gravity of the microcarrier is preferably 1.0 g/cm$^3$ or more, more preferably 1.05 g/cm$^3$ or more, still more preferably 1.1 g/cm$^3$ or more, and preferably 2.0 g/cm$^3$ or less, more preferably 1.5 g/cm$^3$ or less, still more preferably 1.3 g/cm$^3$ or less. When the specific gravity is the lower limit or more, the microcarrier suitably settles, and

recovery efficiency can be enhanced. When the specific gravity is the above upper limit or less, swirability by a stirring blade can be improved.

The specific gravity of the microcarrier is measured using a true specific gravity meter.

An average particle diameter of the microcarrier is preferably 100 um or more, more preferably 150 um or more, still more preferably 200 um or more, even more preferably 250 um or more, particularly preferably 300 um or more, and preferably 1500 um or less, more preferably 1000 um or less, still more preferably 800 um or less, even more preferably 700 um or less, particularly preferably 500 um or less. The average particle diameter of the microcarrier is preferably 100 um or more and 1500 um or less, more preferably 150 um or more and 1000 um or less, still more preferably 200 um or more and 800 um or less, even more preferably 250 um or more and 700 um or less, and particularly preferably 300 um or more and 500 um or less. When the average particle diameter is the above lower limit or more, the cell culture efficiency can be further enhanced. When the average particle diameter is the above upper limit or less, a cell mass can be formed with a further uniform thickness on the surface of each microcarrier. When the average particle diameter is the above upper limit or less, an area to which cells can adhere can be further increased. In the conventional microcarrier, as the average particle diameter is larger, the microcarrier is more likely to be damaged in the cell culture process; however, in the microcarrier according to the present invention, even when the average particle diameter is relatively large, breakage of the microcarrier in the cell culture process can be suppressed.

The particle diameter of the microcarrier means its diameter when the microcarrier has a perfect spherical shape, and when the microcarrier has a shape other than a perfect sphere, the particle diameter of the microcarrier means a diameter when it is assumed that the microcarrier is a perfect sphere corresponding to the volume thereof.

The average particle diameter of the microcarrier is preferably a number average particle diameter. The average particle diameter of the microcarrier is determined by observing arbitrary 50 microcarriers with an electron microscope or an optical microscope, and calculating an average value of the particle diameters of the microcarriers or using a particle size distribution measurement apparatus. In the observation with the electron microscope or the optical microscope, the particle diameter of the microcarrier per particle is determined as a particle diameter corresponding to an equivalent circle diameter. In the observation with the electron microscope or the optical microscope, the average particle diameter obtained from the equivalent circle diameters of arbitrary 50 microcarriers is substantially equal to the average particle diameter corresponding to an equivalent sphere diameter. In the particle size distribution measurement apparatus, the particle diameter of the microcarrier per particle is determined as a particle diameter corresponding to an equivalent sphere diameter. The average particle diameter of the microcarrier is preferably calculated using the particle size distribution measurement apparatus.

A variation coefficient (CV value) of the particle diameter of the microcarrier is preferably 10% or less, more preferably 8% or less, still more preferably 5% or less, and particularly preferably 3% or less. When the variation coefficient (CV value) is the above upper limit or less, uniformity of a sedimentation rate can be enhanced, and the cell culture efficiency can be further enhanced. The variation coefficient (CV value) of the particle diameter of the microcarrier may be 0% or more, 0.1% or more, 0.5% or more, or 1% or more. The variation coefficient (CV value) of the particle diameter of the microcarrier may be 0% or more and 10% or less, 0.1% or more and 8% or less, 0.1% or more and 50 or less, or 1% or more and 3% or less.

The variation coefficient (CV value) of the particle diameter of the microcarrier is calculated as follows.

$$\text{CV value (\%)} = (\rho/Dn) \times 100$$

ρ: Standard deviation of microcarrier particle diameter
Dn: Average particle diameter of microcarrier

Examples of a method of reducing the variation coefficient (CV value) of the particle diameter of the microcarrier include a dry classification method and a wet classification method.

The shape of the microcarrier is not particularly limited. The shape of the microcarrier may be a spherical shape, a shape other than a spherical shape, or a shape such as a flat shape. The spherical shape is not limited to a perfect spherical shape, and includes a substantially spherical shape, for example, a shape having an aspect ratio (major axis/minor axis) of 1.5 or less.

Hereinafter, the present invention will be specifically described with reference to the drawings.

Fig. 1 is a cross-sectional view schematically showing the microcarrier for cell culture according to one embodiment of the present invention.

The microcarrier 1 for cell culture shown in Fig. 1 includes a base particle 2 and a coating layer 3 coating an outer surface of the base particle 2. The coating layer 3 is disposed on a surface of the base particle 2 and is in contact with the surface of the base particle 2. The coating layer 3 coats the entire outer surface of the base particle 2. The microcarrier 1 has a strength at break of 1000 mN or more.

Hereinafter, other details of the microcarrier will be described.
In the present specification, "(meth)acrylate" means one or both of "acrylate" and "methacrylate", and "(meth)acryl" means one or both of "acryl" and "methacryl".

(Base particle)

The material of the base particle is not particularly limited as long as the microcarrier has a strength at break of 1000 mN or more. The material of the base particle may be an organic material, an inorganic material, or both the organic material and the inorganic material. The base particle may contain a resin, an inorganic filler, or both the resin and the inorganic filler, or may not contain a resin. The base particle may be a resin particle or an inorganic particle. The base particle preferably contains a resin. From the viewpoint of further suppressing breakage of the microcarrier in the cell culture process, the base particle is preferably a resin particle. One kind of the materials of the base particle may be used alone, and two or more kinds thereof may be used in combination. One kind of the resins may be used alone, and two or more kinds thereof may be used in combination.
Examples of the resin include polyolefin resins, acrylic resins, polycarbonate, polyamide, phenol formaldehyde resin, melamine formaldehyde resin, benzoguanamine formaldehyde resin, urea formaldehyde resin, phenol resin, melamine resin, benzoguanamine resin, urea resin, epoxy resin, unsaturated polyester resin, saturated polyester resin, polyethylene terephthalate, polysulfone, polyphenylene oxide, polyacetal, polyimide, polyamideimide, polyether ether ketone, polyether sulfone, divinylbenzene polymer, and divinylbenzene copolymer.
When the base particle is a resin particle, among various resins, a resin capable of controlling the strength at break of the microcarrier to 1000 mN or more is suitably used.
The resin is preferably a polymer of a monomer having an ethylenically unsaturated group. The base particle preferably contains a polymer of a monomer having an ethylenically unsaturated group. In this case, the specific gravity and strength of the base particle can be favorably adjusted, so that the specific gravity of the microcarrier can be adjusted to a suitable range, and the strength at break of the microcarrier can be increased.
The monomer having the ethylenically unsaturated group preferably has two or more ethylenically unsaturated groups. The base particle preferably contains a polymer of a monomer having two or more ethylenically unsaturated groups. In this case, the specific gravity and strength of the base particle can be more favorably adjusted, so that the specific gravity of the microcarrier can be adjusted to a suitable range, and the strength at break of the microcarrier can be increased.
Examples of the polymer of the monomer having the ethylenically unsaturated group include an acrylic resin, a divinylbenzene polymer, and a divinylbenzene copolymer. One kind of the monomer having the ethylenically unsaturated group may be used alone, or two or more kinds thereof may be used in combination.
The polymer of the monomer having the ethylenically unsaturated group is preferably an acrylic resin, a divinylbenzene polymer, or a divinylbenzene copolymer. In this case, the specific gravity and strength of the base particle can be favorably adjusted, so that the specific gravity of the microcarrier can be adjusted to a suitable range, and the strength at break of the microcarrier can be increased.
When the base particle contains the polymer of the monomer having the ethylenically unsaturated group, the polymer of the monomer having the ethylenically unsaturated group preferably has a crosslinked structure. In this case, the specific gravity and strength of the base particle can be favorably adjusted, so that the specific gravity of the microcarrier can be adjusted to a suitable range, and the strength at break of the microcarrier can be increased.
Examples of a method of forming the crosslinked structure include the following methods. (1) A method of polymerizing a polymerizable component containing a monomer having two or more ethylenically unsaturated groups. (2) A method of forming a crosslinked structure by reacting a polymer of a monomer having an ethylenically unsaturated group with a crosslinking agent.
In the method (1), examples of the monomer having two or more ethylenically unsaturated groups include divinylbenzene, polyfunctional (meth)acrylate, triallyl(iso)cyanurate, triallyl trimellitate, diallyl phthalate, and diallyl acrylamide. One kind of the monomer having two or more ethylenically unsaturated groups may be used alone, or two or more kinds thereof may be used in combination.
In the method (1), the polymerizable component may contain another monomer having an ethylenically unsaturated group. Examples of another monomer having the ethylenically unsaturated group include styrene, monofunctional (meth)acrylate, (meth)acrylic acid, acrylonitrile, and vinyl chloride. One kind of another monomer having the ethylenically unsaturated group may be used alone, or two or more kinds thereof may be used in combination.
Examples of the polymer obtained by the method (1) include a copolymer of divinylbenzene and styrene and a copolymer of polyfunctional (meth)acrylate and monofunctional (meth)acrylate.
Examples of the method (2) include a method in which a polymerizable component containing a monomer having an ethylenically unsaturated group and a functional group containing active hydrogen in the molecule is polymerized to obtain a polymer, and then the polymers are crosslinked with each other using a crosslinking agent.

Examples of the functional group containing active hydrogen include a hydroxyl group, a carboxyl group, an amino group, and a phenol group. Examples of the monomer having an ethylenically unsaturated group and a functional group containing active hydrogen in the molecule include hydroxyl group-containing (meth)acrylate, (meth)acrylic acid, and amino group-containing (meth)acrylate. One kind of the monomer having an ethylenically unsaturated group and a functional group containing active hydrogen in the molecule may be used alone, or two or more kinds thereof may be used in combination.

The crosslinking agent is not particularly limited as long as it can react with the functional group containing active hydrogen, and examples thereof include a polyfunctional isocyanate compound and a polyfunctional epoxy compound. One kind of the crosslinking agents may be used alone, and two or more kinds thereof may be used in combination.

As long as the strength at break of the resulting microcarrier is 1000 mN or more, a method of forming the crosslinked structure is not limited to the above methods.

The base particle can be obtained, for example, by polymerizing the monomer having the ethylenically unsaturated group. The polymerization method is not particularly limited, and examples thereof include known methods such as radical polymerization, ionic polymerization, polycondensation (condensation polymerization), addition condensation, living polymerization, and living radical polymerization. Other polymerization methods include suspension polymerization in the presence of a radical polymerization initiator.

The base particle may contain an inorganic filler. For example, the specific gravity of the base particle and the microcarrier can be suitably increased by using the inorganic filler and a resin having a small specific gravity in combination.

Examples of the inorganic filler include carbon black, glass filler, and metal filler. One kind of the inorganic filler may be used alone, and two or more kinds thereof may be used in combination.

The content of the resin in 100% by weight of the base particle is preferably 80% by weight or more, more preferably 90% by weight or more, still more preferably 95% by weight or more, even more preferably 97% by weight or more, still even more preferably 99% by weight or more, and most preferably 100% by weight (whole amount). The content of the resin in 100% by weight of the base particle may be 100% by weight or less or less than 100% by weight.

The content of the polymer of the monomer having the ethylenically unsaturated group in 100% by weight of the base particle is preferably 80% by weight or more, more preferably 90% by weight or more, still more preferably 95% by weight or more, even more preferably 97% by weight or more, still even more preferably 99% by weight or more, and most preferably 100% by weight (whole amount). The content of the polymer of the monomer having the ethylenically unsaturated group in 100% by weight of the base particle may be 100% by weight or less or less than 100% by weight.

When the base particle is the inorganic particle, examples of the inorganic particle include graphite particles, glass particles, and metal particles.

The specific gravity of the base particle is preferably 1.0 g/$cm^3$ or more, more preferably 1.05 g/$cm^3$ or more, still more preferably 1.1 g/$cm^3$ or more, and preferably 2.0 g/$cm^3$ or less, more preferably 1.5 g/$cm^3$ or less, still more preferably 1.3 g/$cm^3$ or less, even more preferably 1.2 g/$cm^3$ or less, and particularly preferably 1.15 g/$cm^3$ or less. When the specific gravity is the lower limit or more and the upper limit or less, the specific gravity of the microcarrier can be adjusted to a suitable range.

The specific gravity of the base particle is measured using a true specific gravity meter.

The average particle diameter of the base particle is preferably 100 um or more, more preferably 150 um or more, still more preferably 200 um or more, even more preferably 250 um or more, particularly preferably 300 um or more, and preferably 1500 um or less, more preferably 1000 um or less, still more preferably 800 um or less, even more preferably 700 um or less, particularly preferably 500 um or less. The average particle diameter of the base particle is preferably 100 um or more and 1500 um or less, more preferably 150 um or more and 1000 um or less, still more preferably 200 um or more and 800 um or less, even more preferably 250 um or more and 700 um or less, and particularly preferably 300 um or more and 500 um or less. When the average particle diameter is the above lower limit or more, the cell culture efficiency can be further enhanced. When the average particle diameter is the above upper limit or less, a cell mass can be formed with a further uniform thickness on the surface of each microcarrier. When the average particle diameter is the above upper limit or less, an area to which cells can adhere can be further increased.

The particle diameter of the base particle means its diameter when the base particle has a perfect spherical shape, and when the base particle has a shape other than a perfect sphere, the particle diameter of the base particle means a diameter when it is assumed that the base particle is a perfect sphere corresponding to the volume thereof.

The average particle diameter of the base particle is preferably a number average particle diameter. The average particle diameter of the base particle is determined by observing arbitrary 50 base particles with an electron microscope or an optical microscope, and calculating an average value of the particle diameters of the base particles or using a particle size distribution measurement apparatus. In the observation with the electron microscope or the optical microscope, the particle diameter of the base particle per particle is determined as a particle diameter corresponding to an equivalent circle diameter. In the observation with the electron microscope or the optical microscope, the average particle diameter obtained from the equivalent circle diameters of arbitrary 50 base particles is substantially equal to the average

particle diameter corresponding to an equivalent sphere diameter. In the particle size distribution measurement apparatus, the particle diameter of the base particle per particle is determined as a particle diameter corresponding to an equivalent sphere diameter. The average particle diameter of the base particle is preferably calculated using the particle size distribution measurement apparatus.

(Coating layer)

The microcarrier includes the base particle and the coating layer coating the outer surface of the base particle. The coating layer is a layer including a component different from the component of the base particle. Examples of the component constituting the coating layer include peptides and synthetic resins. The coating layer preferably contains a synthetic resin. One kind of the synthetic resins may be used alone, and two or more kinds thereof may be used in combination.

From the viewpoint of enhancing adhesiveness between the microcarrier and the cell and maintaining a low swelling degree of the microcarrier, the synthetic resin preferably has a polyvinyl alcohol derivative skeleton or a poly(meth)acrylic acid ester skeleton. In this case, the synthetic resin may have a polyvinyl alcohol derivative skeleton, a poly(meth)acrylic acid ester skeleton, or both the polyvinyl alcohol derivative skeleton and the poly(meth)acrylic acid ester skeleton.

From the viewpoint of enhancing the adhesiveness between the microcarrier and the cell, the coating layer preferably contains a peptide moiety, and more preferably contains a synthetic resin having a peptide moiety (peptide skeleton). That is, from the viewpoint of enhancing the adhesiveness between the microcarrier and the cell, the synthetic resin more preferably has a peptide moiety. The aspect in which the coating layer contains a peptide moiety includes not only an aspect in which the coating layer contains a synthetic resin having a peptide moiety but also an aspect in which the coating layer contains only a peptide. From the viewpoint of further enhancing the adhesiveness between the microcarrier and the cell, the synthetic resin preferably has a polyvinyl alcohol derivative skeleton or a poly(meth)acrylic acid ester skeleton, and a peptide moiety.

In the present specification, the "resin having a polyvinyl alcohol derivative skeleton or a poly(meth)acrylic acid ester skeleton, and a peptide moiety" may be described as a "peptide-conjugated resin". The peptide-conjugated resin may have a polyvinyl alcohol derivative skeleton and a peptide moiety, a poly(meth)acrylic acid ester skeleton and a peptide moiety, or a polyvinyl alcohol derivative skeleton, a poly(meth)acrylic acid ester skeleton, and a peptide moiety.

In the peptide-conjugated resin having the polyvinyl alcohol derivative skeleton, the polyvinyl alcohol derivative skeleton and the peptide moiety are preferably bonded to each other via a linker moiety. Therefore, the peptide-conjugated resin having the polyvinyl alcohol derivative skeleton preferably has the polyvinyl alcohol derivative skeleton, the peptide moiety, and the linker moiety.

In the peptide-conjugated resin having the poly(meth)acrylic acid ester skeleton, the poly(meth)acrylic acid ester skeleton and the peptide moiety may be bonded to each other via a linker moiety, or may be directly bonded to each other without the linker moiety. The peptide-conjugated resin having the poly(meth)acrylic acid ester skeleton may have the poly(meth)acrylic acid ester skeleton, the peptide moiety, and the linker moiety.

<Polyvinyl alcohol derivative skeleton>

The polyvinyl alcohol derivative skeleton is a skeleton portion derived from a polyvinyl alcohol derivative. The polyvinyl alcohol derivative is a compound derived from polyvinyl alcohol. From the viewpoint of further enhancing the adhesiveness between the microcarrier and the cell, the polyvinyl alcohol derivative is preferably a polyvinyl acetal resin, and the polyvinyl alcohol derivative skeleton is preferably a polyvinyl acetal skeleton. That is, the synthetic resin preferably has a polyvinyl acetal skeleton and the peptide moiety. Only one kind of each of the polyvinyl alcohol derivative and the polyvinyl acetal resin may be used alone, and two or more kinds thereof may be used in combination.

The polyvinyl alcohol derivative skeleton and the polyvinyl acetal skeleton preferably have an acetal group, a hydroxyl group, and an acetyl group in side chains. However, the polyvinyl alcohol derivative skeleton and the polyvinyl acetal skeleton may not have, for example, an acetyl group. For example, when all of the acetyl groups of the polyvinyl alcohol derivative skeleton and the polyvinyl acetal skeleton are bonded to the linker, the polyvinyl alcohol derivative skeleton and the polyvinyl acetal skeleton may not have an acetyl group.

The polyvinyl acetal resin can be synthesized by acetalizing polyvinyl alcohol with an aldehyde.

The aldehyde used for acetalization of polyvinyl alcohol is not particularly limited. Examples of the aldehyde include an aldehyde having 1 to 10 carbon atoms. The aldehyde may or may not have a chain aliphatic group, a cyclic aliphatic group, or an aromatic group. The aldehyde may be a chain aldehyde or a cyclic aldehyde. One kind of the aldehyde may be used alone, and two or more kinds thereof may be used in combination.

From the viewpoint of further enhancing the adhesiveness between the microcarrier and the cell, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin, the polyvinyl acetal skeleton is more preferably a polyvinyl butyral skeleton, and the synthetic resin more preferably has a polyvinyl butyral skeleton.

In the synthetic resin, an acetalization degree of the polyvinyl alcohol derivative skeleton and the polyvinyl acetal skeleton (butyralization degree in the case of a polyvinyl butyral resin) is preferably 40% by mole or more, more preferably 50% by mole or more, and preferably 90% by mole or less, more preferably 85% by mole or less. When the acetalization degree is the above lower limit or more, the fixability of the cell can be further enhanced, and the cell is efficiently grown. When the acetalization degree is the above upper limit or less, solubility in a solvent can be improved.

In the synthetic resin, a content of the hydroxyl group (hydroxyl group amount) in the polyvinyl alcohol derivative skeleton and the polyvinyl acetal skeleton is preferably 15% by mole or more, more preferably 20% by mole or more, and preferably 45% by mole or less, more preferably 30% by mole or less, still more preferably 25% by mole or less.

In the synthetic resin, an acetylation degree (acetyl group amount) of the polyvinyl alcohol derivative skeleton and the polyvinyl acetal skeleton is preferably 1% by mole or more, more preferably 2% by mole or more, and preferably 5% by mole or less, more preferably 4% by mole or less. When the acetylation degree is the above lower limit or more and the above upper limit or less, reaction efficiency between the polyvinyl acetal resin and the linker can be enhanced.

The acetalization degree, the acetylation degree and the hydroxyl group amount of the polyvinyl alcohol derivative skeleton and the polyvinyl acetal skeleton can be measured by $^1H$-NMR (nuclear magnetic resonance spectrum).

<Poly(meth)acrylic acid ester skeleton>

The poly(meth)acrylic acid ester skeleton is a skeleton portion derived from a poly(meth)acrylic acid ester. The poly(meth)acrylic acid ester is obtained by polymerizing a (meth)acrylic acid ester. The poly(meth)acrylic acid ester skeleton has a skeleton derived from a (meth)acrylic acid ester. Only one kind of the poly(meth)acrylic acid ester may be used alone, or two or more kinds thereof may be used in combination.

Examples of the (meth)acrylic acid ester include (meth)acrylic acid alkyl ester, (meth)acrylic acid cyclic alkyl ester, (meth)acrylic acid aryl ester, (meth)acrylic acid polyethylene glycols, and (meth)acrylic acid phosphorylcholine. Only one kind of the (meth)acrylic acid ester may be used alone, or two or more kinds thereof may be used in combination.

Examples of the (meth)acrylic acid alkyl ester include methyl(meth)acrylate, ethyl(meth)acrylate, n-propyl(meth)acrylate, isopropyl(meth)acrylate, n-butyl(meth)acrylate, isobutyl(meth)acrylate, t-butyl(meth)acrylate, n-octyl(meth)acrylate, isooctyl(meth)acrylate, 2-ethylhexyl(meth)acrylate, nonyl(meth)acrylate, isononyl(meth)acrylate, decyl(meth)acrylate, isodecyl(meth)acrylate, lauryl(meth)acrylate, stearyl(meth)acrylate, and isotetradecyl(meth)acrylate.

The (meth)acrylic acid alkyl ester may be substituted with a substituent such as an alkoxy group having 1 to 3 carbon atoms and a tetrahydrofurfuryl group. Examples of such (meth)acrylic acid alkyl ester include methoxyethyl acrylate and tetrahydrofurfuryl acrylate.

Examples of the (meth)acrylic acid cyclic alkyl ester include cyclohexyl(meth)acrylate and isobornyl(meth)acrylate.

Examples of the (meth)acrylic acid aryl ester include phenyl(meth)acrylate and benzyl(meth)acrylate.

Examples of the (meth)acrylic acid polyethylene glycols include methoxy-polyethylene glycol(meth)acrylate, ethoxy-polyethylene glycol(meth)acrylate, hydroxy-polyethylene glycol(meth)acrylate, methoxy-diethylene glycol(meth)acrylate, ethoxy-diethylene glycol(meth)acrylate, hydroxy-diethylene glycol(meth)acrylate, methoxy-triethylene glycol(meth)acrylate, ethoxy-triethylene glycol(meth)acrylate, and hydroxy-triethylene glycol(meth)acrylate.

Examples of the phosphorylcholine(meth)acrylate include 2-(meth)acryloyloxyethyl phosphorylcholine.

The synthetic resin preferably has a structural unit derived from the (meth)acrylate compound (A) represented by the following formula (A1) or the following formula (A2). The poly(meth)acrylic acid ester skeleton preferably has a structural unit derived from the (meth)acrylate compound (A) represented by the following formula (A1) or the following formula (A2). Thereby, the hydrophobicity of the coating layer can be increased, and therefore the water absorption rate of the microcarrier can be further reduced. Thus, variations in the initial fixing rate after cell seeding can be reduced, and the cells are less likely to be detached from the microcarrier in the medium. The (meth)acrylate compound (A) may contain a (meth)acrylate compound represented by the following formula (A1), may contain a (meth)acrylate compound represented by the following formula (A2), or may contain both the (meth)acrylate compound represented by the following formula (A1) and the (meth)acrylate compound represented by the following formula (A2). When the (meth)acrylate compound (A) contains both the (meth)acrylate compound represented by the following formula (A1) and the (meth)acrylate compound represented by the following formula (A2), R in the following formula (A1) and R in the following formula (A2) may be the same or different. Only one kind of the (meth) acrylate compound (A) may be used alone, or two or more kinds thereof may be used in combination. Each of the (meth)acrylate compound represented by the following formula (A1) and the (meth)acrylate compound represented by the following formula (A2) may be used alone, or two or more kinds thereof may be used in combination.

[Chemical 1]

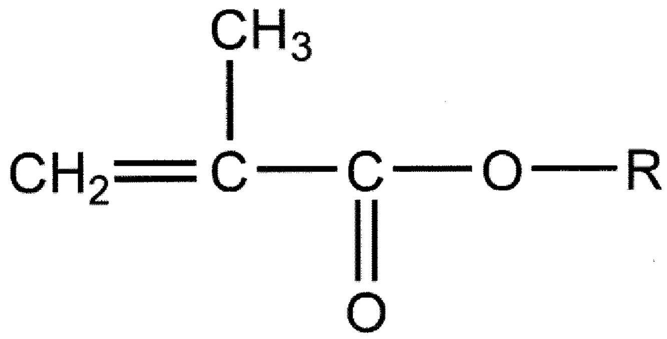

•••(A1)

In the above formula (A1), R represents a hydrocarbon group having 2 or more and 18 or less carbon atoms.

[Chemical 2]

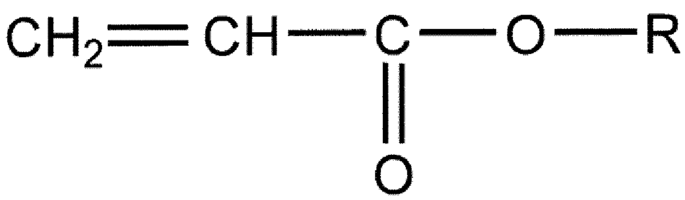

•••(A2)

In the above formula (A2), R represents a hydrocarbon group having 2 or more and 18 or less carbon atoms.

R in the above formula (A1) and R in the above formula (A2) may each be an aliphatic hydrocarbon group or an aromatic hydrocarbon group. From the viewpoint of improving the solubility of the synthetic resin, each of R in the formula (A1) and R in the formula (A2) is preferably an aliphatic hydrocarbon group. The aliphatic hydrocarbon group may be linear, may have a branched structure, may have a double bond, or may not have a double bond. R in the formula (A1) and R in the formula (A2) may each be an alkyl group or an alkylene group.

The number of carbon atoms of R in the formula (A1) and the number of carbon atoms of R in the formula (A2) are each preferably 4 or more, more preferably 6 or more, still more preferably 8 or more, particularly preferably 10 or more, and preferably 16 or less, more preferably 14 or less, and most preferably 12. When the number of carbon atoms is the above lower limit or more, the hydrophobicity of the synthetic resin can be further increased, and therefore the water absorption rate of the microcarrier can be further decreased. When the number of carbon atoms is the above upper limit or less, coatability when the material of the coating layer is disposed on the surface of the base particle can be enhanced. In particular, when the number of carbon atoms is 12, the water absorption rate of the microcarrier can be further reduced, and the coatability can be further enhanced.

The (meth)acrylic acid alkyl ester is preferably the (meth)acrylate compound (A).

A synthetic resin having the poly(meth)acrylic acid ester skeleton may have a skeleton derived from a monomer other than the (meth)acrylic acid ester.

Examples of the monomer other than the (meth)acrylic acid ester include (meth)acrylamides and vinyl compounds. Only one kind of the monomer other than the (meth) acrylic acid ester may be used alone, or two or more kinds thereof may be used in combination.

Examples of the (meth)acrylamides include (meth)acrylamide, N-isopropyl(meth)acrylamide, N-tert-butyl(meth)acrylamide, N,N'-dimethyl(meth)acrylamide, (3-(meth)acrylamidopropyl)trimethylammonium chloride, 4-(meth)acryloylmorpholine, 3-(meth)acryloyl-2-oxazolidinone, N-[3-(dimethylamino)propyl](meth)acrylamide, N-(2-hydroxyethyl)(meth)acrylamide, N-methylol(meth)acrylamide, and 6-(meth)acrylamidohexanoic acid.

Examples of the vinyl compound include ethylene, allylamine, vinylpyrrolidone, maleic anhydride, maleimide, itaconic acid, (meth)acrylic acid, and vinylamine.

<Peptide moiety>

The peptide moiety is a structural moiety derived from a peptide. The peptide moiety has an amino acid sequence. The peptide constituting the peptide moiety may be an oligopeptide or a polypeptide. One kind of the peptide may be used alone, and two or more kinds thereof may be used in combination.

The number of amino acid residues in the peptide moiety is preferably 3 or more, more preferably 4 or more, still more preferably 5 or more, and preferably 10 or less, more preferably 8 or less, still more preferably 6 or less. When the number of the amino acid residues is the above lower limit or more and the above upper limit or less, the adhesiveness to cells after seeding can be further enhanced, and a proliferation rate of cells can be further enhanced. However, the number of the amino acid residues in the peptide moiety may be more than 10 or more than 15.

The peptide moiety preferably has a cell-adhesive amino acid sequence. The cell-adhesive amino acid sequence refers to an amino acid sequence whose cell adhesion activity has been confirmed by a phage display method, a sepharose beads method, or a plate coating method. As the phage display method, for example, a method described in "The Journal of Cell Biology, Volume 130, Number 5, September 1995 1189-1196" can be used. As the sepharose beads method, for example, a method described in "Protein, Nucleic Acid and Enzyme, Vol. 45 No. 15 (2000) 2477" can be used. As the plate coating method, for example, a method described in "Protein, Nucleic Acid and Enzyme, Vol. 45 No. 15 (2000) 2477" can be used.

Examples of the cell-adhesive amino acid sequence include RGD sequence (Arg-Gly-Asp), YIGSR sequence (Tyr-Ile-Gly-Ser-Arg), PDSGR sequence (Pro-Asp-Ser-Gly-Arg), HAV sequence (His-Ala-Val), ADT sequence (Ala-Asp-Thr), QAV sequence (Gln-Ala-Val), LDV sequence (Leu-Asp-Val), IDS sequence (Ile-Asp-Ser), REDV sequence (Arg-Glu-Asp-Val), IDAPS sequence (Ile-Asp-Ala-Pro-Ser), KQAGDV sequence (Lys-Gln-Ala-Gly-Asp-Val), and TDE sequence (Thr-Asp-Glu). In addition, examples of the cell-adhesive amino acid sequence include sequences described in "Medicina Philosophica, Vol. 9, No. 7, pp. 527-535, 1990" and "Journal of Osaka Women's and Children's Hospital, Vol. 8, No. 1, pp. 58-66, 1992", and the like. The peptide moiety may have only one type of cell-adhesive amino acid sequence, or may have two or more types.

The cell-adhesive amino acid sequence preferably has at least one of the above-mentioned cell-adhesive amino acid sequences, more preferably has at least an RGD sequence, a YIGSR sequence or a PDSGR sequence, and further preferably has at least an RGD sequence represented by the following formula (1). In this case, the adhesiveness to cells after seeding can be further enhanced, and the proliferation rate of cells can be further enhanced.

Arg-Gly-Asp-X ··· Formula (1)

In the above formula (1), X represents Gly, Ala, Val, Ser, Thr, Phe, Met, Pro, or Asn.

The peptide moiety may be linear or may have a cyclic peptide skeleton. From the viewpoint of further enhancing cell proliferation, the peptide moiety preferably has a cyclic peptide skeleton. The cyclic peptide skeleton is a cyclic skeleton composed of a plurality of amino acids. From the viewpoint of more effectively exhibiting the effects of the present invention, the cyclic peptide skeleton is preferably composed of four or more amino acids, more preferably composed of five or more amino acids, and preferably composed of ten or less amino acids.

In the peptide-conjugated resin, the content of the peptide moiety is preferably 0.1% by mole or more, more preferably 1% by mole or more, still more preferably 5% by mole or more, particularly preferably 10% by mole or more, and preferably 60% by mole or less, more preferably 50% by mole or less, still more preferably 35% by mole or less, particularly preferably 25% by mole or less. When the content of the peptide moiety is the above lower limit or more, the adhesiveness to cells after seeding can be further enhanced, and the proliferation rate of cells can be further enhanced. When the content of the peptide moiety is the above upper limit or less, the production cost can be suppressed. The content (% by mole) of the peptide moiety is a substance amount of the peptide moiety with respect to a sum of substance amounts of the respective structural units constituting the peptide-conjugated resin.

The content of the peptide moiety can be measured by, for example, NMR, FT-IR, or LC-MS.

<Linker moiety>

The linker moiety is a structural moiety derived from a linker. The linker moiety is usually located between the polyvinyl alcohol derivative skeleton or the poly(meth)acrylic acid ester skeleton and the peptide moiety. The polyvinyl alcohol derivative skeleton or the poly(meth)acrylic acid ester skeleton and the peptide moiety are bonded via the linker moiety. The linker moiety is formed by the linker (crosslinking agent). One kind of the linkers may be used alone, and two or more kinds thereof may be used in combination.

The linker is preferably a compound having a functional group capable of bonding to the peptide, and more preferably a compound having a functional group capable of condensing with a carboxyl group or an amino group of the peptide.

Examples of the functional group capable of condensing with a carboxyl group or an amino group of the peptide include a carboxyl group, a thiol group, an amino group, a hydroxyl group, and a cyano group.
From the viewpoint of favorably reacting with the peptide, the linker is preferably a compound having a carboxyl group or an amino group, and more preferably a compound having a carboxyl group.
In the case of obtaining a peptide-conjugated resin having a polyvinyl alcohol derivative skeleton, examples of the carboxyl group-containing linker include (meth)acrylic acid and carboxyl group-containing acrylamide. By using a carboxylic acid (carboxylic acid monomer) having a polymerizable unsaturated group as the linker having a carboxyl group, the carboxylic acid monomer can be polymerized by graft polymerization at the time of introduction of the linker, so that the number of carboxyl groups that can react with the peptide can be increased.
From the viewpoint of favorably bonding the polyvinyl alcohol derivative and the peptide, the linker is preferably(meth)acrylic acid, and more preferably acrylic acid.
In the case of obtaining a peptide-conjugated resin having a poly(meth)acrylic acid ester skeleton, the linker preferably has a functional group capable of bonding to a (meth)acrylic acid ester. Examples of the functional group capable of bonding to the (meth)acrylic acid ester include a vinyl group, a (meth)acryloyl group, and an allyl group. The linker more preferably has a (meth) acryloyl group as the functional group capable of bonding to the (meth)acrylic acid ester, and is preferably a compound having a carboxyl group or an amino group and having a (meth)acryloyl group.
Examples of the linker in the case of obtaining the peptide-conjugated resin having a poly(meth)acrylic acid ester skeleton include (meth)acrylic acid, itaconic acid, and acrylamide.
From the viewpoint of favorably bonding the poly(meth)acrylic acid ester and the peptide, the linker is preferably (meth)acrylic acid or itaconic acid, and more preferably (meth)acrylic acid.

<Other details of coating layer>

The weight average molecular weight of the synthetic resin is preferably 10,000 or more, more preferably 50,000 or more, and preferably 1.2 million or less, more preferably 600,000 or less. When the weight average molecular weight is the above lower limit or more and the above upper limit or less, the effect of the present invention can be more effectively exhibited. When the weight average molecular weight is the above upper limit or less, extensibility of cells in cell culture can be more effectively enhanced.
The weight average molecular weight can be measured by, for example, the following method. The synthetic resin is dissolved in tetrahydrofuran (THF) to prepare a 0.2% by weight solution of the synthetic resin. Next, evaluation is performed under the following measurement conditions using a gel permeation chromatography (GPC) measuring device (APC system, manufactured by Waters Corporation).

Column: HSPgel HR MB-M 6.0 × 150 mm
Flow rate: 0.5 mL/min
Column temperature: 40°C
Injection volume: 10 μL
Detector: RI, PDA
Standard sample: Polystyrene

The coating layer may contain only the resin having a polyvinyl alcohol derivative skeleton or a poly(meth)acrylic acid ester skeleton. The coating layer may contain the resin having a polyvinyl alcohol derivative skeleton or a poly(meth)acrylic acid ester skeleton and not having a peptide moiety, and the peptide-conjugated resin. The coating layer may contain other components such as a resin having neither the polyvinyl alcohol derivative skeleton nor the poly(meth)acrylic acid ester skeleton. Examples of the other component include a polyolefin resin, a polyether resin, polyester, an epoxy resin, a polyamide resin, a polyimide resin, a polyurethane resin, a polycarbonate resin, cellulose, and a polypeptide. One kind of the other components may be used alone, and two or more kinds thereof may be used in combination.
The coating layer may have only a layer (hereinafter, may be referred to as "layer X") containing the resin having a polyvinyl alcohol derivative skeleton or a poly(meth)acrylic acid ester skeleton. The coating layer may have a layer (hereinafter, may be referred to as "layer Y") containing no resin having a polyvinyl alcohol derivative skeleton or a poly(meth)acrylic acid ester skeleton. The coating layer may have the layer X and the layer Y. When the coating layer has the layer X and the layer Y, it is preferable that the layer Y is located on the base particle side and the layer X is located outside the layer Y in the coating layer. In this case, the adhesiveness between the microcarrier and the cell can be further enhanced.
The peptide-conjugated resin is preferably present at least on the outer surface of the microcarrier. An outermost layer of the microcarrier is preferably a layer containing the peptide-conjugated resin. In this case, the adhesiveness between the microcarrier and the cell can be further enhanced.

The content of the synthetic resin in 100% by weight of the coating layer is preferably 90% by weight or more, more preferably 95% by weight or more, still more preferably 97.5% by weight or more, particularly preferably 99% by weight or more, and most preferably 100% by weight (whole amount). When the content of the synthetic resin is the above lower limit or more, the effect of the present invention can be more effectively exhibited. The content of the synthetic resin in 100% by weight of the coating layer may be 100% by weight or less or less than 100% by weight.

The content of the resin having a polyvinyl alcohol derivative skeleton or a poly(meth)acrylic acid ester skeleton or the peptide-conjugated resin in 100% by weight of the coating layer is preferably 90% by weight or more, more preferably 95% by weight or more, still more preferably 97.5% by weight or more, particularly preferably 99% by weight or more, and most preferably 100% by weight (whole amount). When the content of the resin having a polyvinyl alcohol derivative skeleton or a poly(meth)acrylic acid ester skeleton or the peptide-conjugated resin is the above lower limit or more, the effects of the present invention can be more effectively exhibited. The content of the resin having a polyvinyl alcohol derivative skeleton or a poly(meth)acrylic acid ester skeleton or the peptide-conjugated resin in 100% by weight of the coating layer may be 100% by weight or less, or may be less than 100% by weight.

A surface area (coverage) coated with the coating layer in 100% of a total surface area of the base particle is preferably 50% or more, more preferably 70% or more, still more preferably 90% or more, even more preferably 95% or more, particularly preferably 99% or more, and most preferably 100%. When the coverage is the above lower limit or more, the adhesiveness between the microcarrier and the cell can be further enhanced, and the effect of the present invention can be more effectively exhibited. The coverage may be 100% or less, less than 100%, or 99% or less.

The coverage is determined by observing the microcarrier with an electron microscope or an optical microscope and calculating a percentage of the surface area coated with the coating layer with respect to a projected area of the base particle.

The thickness of the coating layer is preferably 10 nm or more, more preferably 50 nm or more, and preferably 1000 nm or less, more preferably 500 nm or less. When the thickness of the coating layer is the above lower limit or more and the above upper limit or less, the adhesion between the microcarrier and the cell can be further enhanced. Furthermore, when the thickness of the coating layer is the above lower limit or more and the above upper limit or less, the effect of the present invention can be more effectively exhibited.

The thickness of the coating layer can be measured, for example, by observing a cross section of the microcarrier using a scanning electron microscope (SEM). With respect to the thickness of the coating layer, it is preferable to calculate an average value of the thicknesses of arbitrary five positions of the coating layer as the thickness of the coating layer of one microcarrier, and it is more preferable to calculate an average value of the thickness of the entire coating layer as the thickness of the coating layer of one microcarrier. The thickness of the coating layer is preferably determined by calculating an average value of the thickness of the coating layer of each of arbitrary 50 microcarriers.

Examples of a method for obtaining the peptide-conjugated resin having a polyvinyl alcohol derivative skeleton include the following methods.

A polyvinyl alcohol derivative (for example, a polyvinyl acetal resin) is reacted with a linker to obtain a reaction product in which the polyvinyl acetal resin and the linker are bonded. The obtained reactant is reacted with a peptide to obtain a peptide-conjugated resin having a polyvinyl alcohol derivative skeleton (polyvinyl acetal skeleton).

Examples of a method for obtaining the peptide-conjugated resin having a poly(meth)acrylic acid ester skeleton include the following methods.

An acrylic resin obtained by polymerizing a monomer containing a (meth)acrylic acid ester is obtained. The obtained acrylic resin, a peptide, and a linker used as necessary are reacted to obtain a peptide-conjugated resin having a poly(meth)acrylic acid ester skeleton.

Examples of a method for obtaining the peptide-conjugated resin having the polyvinyl alcohol derivative skeleton and the poly(meth)acrylic acid ester skeleton include the following methods.

A resin having a polyvinyl alcohol derivative skeleton and a poly(meth)acrylic acid ester skeleton is obtained by the following method (i), (ii), or (iii). (i) A polyvinyl acetal resin is synthesized using polyvinyl alcohol copolymerized with an acrylic acid ester. (ii) A polyvinyl acetal resin is synthesized using polyvinyl alcohol and polyvinyl alcohol copolymerized with an acrylic acid ester. (iii) An acrylic acid ester is graft-copolymerized with a polyvinyl acetal resin. A resin obtained by the method (i), (ii) or (iii), a peptide, and a linker used as necessary are reacted to obtain a peptide-conjugated resin having the polyvinyl alcohol derivative skeleton and the poly(meth)acrylic acid ester skeleton.

Examples of a method for obtaining a microcarrier by disposing the coating layer on the surface of the base particle include the following methods (1) and (2).

Method (1): The peptide-conjugated resin obtained by the above-described method is dissolved in a solvent to obtain a solution containing the peptide-conjugated resin. By spraying the solution containing the peptide-conjugated resin onto the base particle or separating the base particle impregnated with the solution containing the peptide-conjugated resin, a microcarrier including a layer (coating layer) containing the peptide-conjugated resin on the outer surface of the base particle can be produced.

Method (2): A resin (resin before peptide bond) having a polyvinyl alcohol derivative skeleton or a poly(meth)acrylic acid ester skeleton is provided. This resin is dissolved in a solvent to obtain a resin-containing solution. The resin-containing solution is sprayed onto the base particle or the base particle impregnated with the resin-containing solution is separated to obtain a particle in which a layer containing a resin having a polyvinyl alcohol derivative or a poly(meth)acrylic acid ester is disposed on the outer surface of the base particle. The obtained particles are reacted with a resin containing the polyvinyl alcohol derivative or the poly(meth)acrylic acid ester contained in the layer, a peptide, and a linker used as necessary by the above-described method. In this way, a microcarrier including a layer containing a resin having a polyvinyl alcohol derivative skeleton or a poly(meth)acrylic acid ester skeleton and not having a peptide moiety and a layer containing a peptide-conjugated resin as the coating layers on the outer surface of the base particle can be produced.

(Other details of microcarrier)

The microcarrier is used for culturing a cell.

Examples of the cells include animal cells such as human cells, mouse cells, rat cells, pig cells, cow cells, and monkey cells. In addition, examples of the cells include somatic cells, and examples thereof include stem cells, progenitor cells, and mature cells. The somatic cells may be cancer cells.

Examples of the stem cells include mesenchymal stem cells (MSCs), iPS cells, ES cells, Muse cells, embryonic cancer cells, embryonic germ cells, and mGS cells.

Examples of the mature cells include nerve cells, cardiomyocytes, retinal cells, and hepatocytes.

The microcarrier is preferably used for three-dimensional culture of cells. The three-dimensional culture is a culture method in which cells are cultured with a thickness also in a longitudinal direction with respect to two-dimensional culture in which cells are cultured on a plane such as a plate.

The microcarrier is preferably used for serum-free medium culture. In the microcarrier, even in the serum-free culture medium culture containing no feeder cell or adhesive protein, cell adhesiveness can be enhanced, and in particular, the initial fixing rate after cell seeding can be further enhanced. In the microcarrier, the effect of the present invention can be exhibited even in the serum-free culture medium culture. In particular, when the coating layer contains the peptide-conjugated resin, even in the serum-free culture medium culture, the effect of increasing the cell adhesiveness and the effect of increasing the initial fixing rate after cell seeding are effectively exhibited.

The microcarrier preferably does not substantially contain animal-derived raw materials. By not containing the animal-derived raw materials, it is possible to provide a microcarrier having high safety and little variation in quality at the time of production. The phrase "does not substantially contain animal-derived raw materials" means that the animal-derived raw materials in the microcarrier are 3% by weight or less. In the microcarrier, the animal-derived raw material in the microcarrier is preferably 1% by weight or less, and most preferably 0% by weight. That is, it is most preferable that the microcarrier does not contain any animal-derived raw material.

(Cell culture method)

Cells can be cultured using the microcarrier. The cell culture method according to the present invention is a cell culture method using the microcarrier described above. Examples of the cells include the cells described above.

The cell culture method preferably includes a step of adhering the cell to the microcarrier. The cell may be a cell mass. The cell mass can be obtained by adding a cell detachment agent to a confluent culture container and uniformly performing crushing by pipetting. The cell detachment agent is not particularly limited, but is preferably an ethylenediamine/phosphate buffer solution. The size of the cell mass is preferably 50 um to 200 um.

Hereinafter, the present invention will be described in more detail with reference to Examples and Comparative Examples. The present invention is not limited only to these examples.

The content of the structural unit in the obtained resin was measured by $^1$H-NMR (nuclear magnetic resonance spectrum) after dissolving the synthetic resin in DMSO-d6 (dimethyl sulfoxide).

(Example 1)

(1) Preparation of base particle A

Divinylbenzene (purity: 57%) (800 parts by weight) and styrene (200 parts by weight) were mixed to obtain a mixed solution. To the resulting mixed solution, 20 parts by weight of benzoyl peroxide was added, and the resulting mixture was stirred until it was uniformly dissolved to obtain a monomer mixed solution. In a reaction kettle, 4000 parts by weight of a 2% by weight aqueous solution prepared by dissolving polyvinyl alcohol having a molecular weight of about 1700 in pure water were placed. Subsequently, the obtained monomer mixed solution was put in a reaction kettle and stirred for 4 hours to adjust the particle diameter so that monomer droplets would have a predetermined particle

diameter. Then, the reaction was carried out in a nitrogen atmosphere at 85°C for 9 hours, and a polymerization reaction of monomer droplets was carried out to obtain particles. The obtained particles were washed with hot water, methanol, and acetone several times each, and then subjected to classification operation to obtain base particles A having an average particle diameter of 100 um and a CV value of particle diameter of 1%. The base particle A is a resin particle of a divinylbenzene copolymer (described as DVB in the table).

(2) Preparation of polyvinyl acetal resin

Into a reactor equipped with a stirrer, 2700 mL of ion-exchanged water, 300 parts by weight of polyvinyl alcohol having an average polymerization degree of 1700 and a saponification degree of 99% by mole were charged, and heated and dissolved with stirring to obtain a solution. To the obtained solution, 35% by weight hydrochloric acid was added as a catalyst so that the hydrochloric acid concentration was 0.2% by weight. Then, the temperature was adjusted to 15°C, and 22 parts by weight of n-butyraldehyde was added while stirring. Next, 148 parts by weight of n-butyraldehyde was added to precipitate a white particulate polyvinyl acetal resin (polyvinyl butyral resin). Fifteen minutes after precipitation, 35% by weight hydrochloric acid was added so that the hydrochloric acid concentration was 1.8% by weight, then heated to 50°C, and held at 50°C for 2 hours. Then, the solution was cooled and neutralized, and then the polyvinyl butyral resin was washed with water and dried to obtain a polyvinyl acetal resin (polyvinyl butyral resin, average polymerization degree 1700, acetalization degree (butyralization degree) 70% by mole, hydroxyl group amount 27% by mole, acetylation degree 3% by mole).

(3) Formation of linker moiety

The obtained polyvinyl acetal resin (99 parts by weight) and 1 part by weight of acrylic acid (linker) were dissolved in 300 parts by weight of THF (tetrahydrofuran), and reacted under ultraviolet irradiation for 20 minutes in the presence of a photoradical polymerization initiator to graft copolymerize the polyvinyl acetal resin and the acrylic acid, thereby forming a linker moiety.

(4) Preparation of polyvinyl acetal resin-coated particles formed with linker moiety

The polyvinyl acetal resin (1 part by weight) formed with the linker moiety was dissolved in butanol (19 parts by weight). The base particle A (1 part by weight) was added to the obtained solution, and the mixture was stirred, then filtered, washed with pure water, and vacuum-dried at 60°C for 5 hours to obtain a polyvinyl acetal resin-coated particle formed with the linker moiety.

(5) Preparation of microcarrier

A linear peptide having an amino acid sequence of Gly-Arg-Gly-Asp-Ser (five amino acid residues) was provided. This peptide (1 part by weight) and 1-ethyl-3-(3-dimethylaminopropyl)carbodiimide hydrochloride (condensing agent) (1 part by weight) were added to phosphate buffered saline containing neither calcium nor magnesium so that the final concentration of the peptide was 1 mM to prepare a peptide-containing solution. To 20 parts by weight of the obtained peptide-containing liquid was added 1 part by weight of polyvinyl acetal resin-coated particles formed with a linker moiety, so that the carboxyl group of the linker moiety and the amino group of Gly of the peptide were dehydration-condensed. The resulting suspension was filtered, washed with pure water, and vacuum-dried at 60°C for 5 hours to obtain a microcarrier. In the table, the peptide-conjugated resin having a polyvinyl alcohol derivative skeleton (polyvinyl acetal skeleton) obtained by the above-described method is described as a resin X1. The resin X1 has an amino acid sequence of Gly-Arg-Gly-Asp-Ser as the peptide moiety.

(Example 2)

(1) Preparation of base particle B

A polymerization reaction was performed in the same manner as in Example 1 to obtain particles. The obtained particles were subjected to a classification operation to obtain base particles B having an average particle diameter of 200 um and a CV value of particle diameter of 1%.

(2) Preparation of microcarrier

A microcarrier was prepared in the same manner as in Example 1, except that the obtained base particle B

was used.

(Example 3)

(1) Preparation of base particle C

A polymerization reaction was performed in the same manner as in Example 1 to obtain particles. The obtained particles were subjected to a classification operation to obtain base particles C having an average particle diameter of 300 um and a CV value of particle diameter of 1%.

(2) Preparation of microcarrier

A microcarrier was prepared in the same manner as in Example 1, except that the obtained base particle C was used.

(Example 4)

(1) Production of base particle D

A polymerization reaction was performed in the same manner as in Example 1 to obtain particles. The obtained particles were subjected to a classification operation to obtain base particles D having an average particle diameter of 400 um and a CV value of particle diameter of 1%.

(2) Preparation of microcarrier

A microcarrier was prepared in the same manner as in Example 1, except that the obtained base particle D was used.

(Example 5)

(1) Production of base particle E

Particles were obtained in the same manner as in Example 1, except that the blending amount of divinylbenzene was changed from 800 parts by weight to 30 parts by weight, and the blending amount of styrene was changed from 200 parts by weight to 970 parts by weight. The obtained particles were subjected to a classification operation to obtain base particles E having an average particle diameter of 300 um and a CV value of particle diameter of 5%. The base particle E is a resin particle of a polystyrene-divinylbenzene copolymer (described as PS 97%/DVB 3% in the table).

(2) Preparation of microcarrier

A microcarrier was prepared in the same manner as in Example 1, except that the obtained base particle E was used.

(Example 6)

The base particle C was used as the base particle.

(2) Preparation of acrylic resin

Dodecyl acrylate (10 parts by weight) and acrylic acid (2.7 parts by weight) were dissolved in tetrahydrofuran (27 parts by weight) to obtain an acrylic monomer solution. Irgacure 184 (manufactured by BASF SE) (0.0575 parts by weight) was dissolved in the obtained acrylic monomer solution, and the resulting solution was applied onto a PET film. The applied product was irradiated with light having a wavelength of 365 nm at an integrated light amount of 2000 $mJ/cm^2$ using a UV conveyor device ("ECS 301 G1" manufactured by EYE GRAPHICS Co., Ltd.) at 25°C to obtain a (meth)acrylic copolymer solution. The obtained (meth)acrylic copolymer solution was vacuum-dried at 80°C for 3 hours to obtain an acrylic resin having a linker moiety.

(3) Preparation of acrylic resin-coated particles

The obtained acrylic resin (1 part by weight) was dissolved in butanol (19 parts by weight). The base particle (1 part by weight) was added to that solution, and the mixture was stirred, then filtered, washed with pure water, and vacuum-dried at 60°C for 5 hours to obtain acrylic resin-coated particles.

(4) Preparation of microcarrier

The obtained acrylic resin-coated particles were used. A cyclic peptide having an amino acid sequence of Arg-Gly-Asp-Phe-Lys (five amino acid residues, Arg and Lys were bonded to form a cyclic skeleton, and Phe was a D-form) was provided as a peptide. A microcarrier was obtained in the same manner as in Example 1, except that the carboxyl group in the structural unit derived from acrylic acid of the acrylic resin and the amino group of Lys of the peptide were subjected to dehydration condensation using this peptide. In the table, the peptide-conjugated resin having a poly(meth)acrylic acid ester skeleton obtained by the above-described method is described as a resin X2. The resin X2 has an amino acid sequence of Arg-Gly-Asp-Phe-Lys (cyclic peptide skeleton) as the peptide moiety.

(Example 7)

(1) Production of base particle F

Micropearl GS-L 300 (manufactured by Sekisui Chemical Co., Ltd., average particle diameter: 300 um, CV value of particle diameter: 7%, polyfunctional acrylic resin particles) was provided. The particles were subjected to a classification operation to obtain base particles F having an average particle diameter of 300 um and a CV value of particle diameter of 1%. The base particle F is a resin particle of an acrylic resin (described as ACR in the table).

(2) Preparation of microcarrier

A microcarrier was prepared in the same manner as in Example 1, except that the obtained base particle F was used.

(Comparative Example 1)

(1) Production of base particle G

A particle obtained by performing a classification operation on "untreated microcarrier" (the base particle was a polystyrene particle, and was described as PS in the table) manufactured by Corning Incorporated and setting the CV value of the particle diameter to 1% was used as the base particle G.

(2) Preparation of microcarrier

A microcarrier was prepared in the same manner as in Example 1, except that the base particle G was used.

(Comparative Example 2)

The obtained base particle C itself was used as the microcarrier.

(Evaluation)

(1) Specific gravity of base particle and microcarrier

The specific gravities of the obtained base particles and microcarrier were measured in a dry state under an argon gas atmosphere using a true specific gravity meter ("Accupic II" manufactured by Shimadzu Corporation).

(2) Average particle diameter of microcarrier and variation coefficient of particle diameter (CV value)

The obtained microcarrier was observed with a scanning electron microscope. The average particle diameter and the variation coefficient (CV value) of the particle diameter obtained from the equivalent circle diameters of arbitrary 50 microcarriers were calculated.

(3) Thickness of coating layer

A cross section of the obtained microcarrier was observed with a scanning electron microscope. The thickness of the coating layer was measured for each of arbitrary 50 microcarriers, and the average value thereof was taken as the thickness of the coating layer of microcarrier.

(4) Strength at break of microcarrier

The strength at break of the obtained microcarrier was measured by the method described above. As the micro strength evaluation tester, "Microautograph MST-I" manufactured by Shimadzu Corporation was used. In the table, "> 2000" means that the microcarrier did not have a strength at break of 2000 mN or less, that is, the strength at break of the microcarrier exceeded 2000 mN.

(5) Load at inflection point of compression displacement curve

As for the obtained microcarrier, a compression test was performed by the method described above. The load value (mN) and the compressive displacement (um) at that time were measured, and the compression displacement curve indicating the relationship between the compressive displacement (x-axis) and the load value (y-axis) was created. The inflection point of the obtained compression displacement curve was obtained, and a load at the inflection point was calculated. When the compression displacement curve had no inflection point at a load of 700 mN or less, the case was indicated by "-" in the table.

(6) Water absorption rate of microcarrier

The obtained microcarrier was dried in an oven at 100°C for 8 hours. The microcarrier (100.0 mg) was weighed and left to stand for 24 hours in an environment of a temperature of 37°C and a relative humidity of 95% RH. The weight of the microcarrier after being left to stand was measured, and the water absorption rate of the microcarrier was calculated by the following formula.

$$\text{Water absorption rate (\% by weight)} = (W_2 - W_1)/W_1 \times 100$$

$W_1$: Weight of microcarrier before being left to stand (mg)
$W_2$: Weight of microcarrier after being left to stand (mg)

(7) Evaluation of cell culture (cell number)

A 12 well plate (manufactured by Corning Incorporated, flat bottom, not treated) was used as a culture plate. In addition, the following liquid medium and a ROCK (Rhobinding kinase) -specific inhibitor were provided.

STEMFIT medium (manufactured by Ajinomoto Healthy Supply Co., Inc.)

ROCK-Inhibitor (Y27632)

h-iPS cells 201B7 ($3.8 \times 10^4$ cells) in a confluent state and 1 mL of a 0.5 mM ethylenediaminetetraacetic acid/phosphate buffer solution were added to a φ 35 mm dish, and the mixture was allowed to stand at room temperature for 5 minutes. The ethylenediaminetetraacetic acid/phosphate buffer solution was removed, and then pipetted with 1 mL of the liquid medium to obtain a cell suspension. The whole amount of the obtained cell suspension was added to a culture plate containing the obtained microcarrier (60 $cm^2$ in terms of surface area) and 4 mL of a liquid medium. The culture plate was placed in a shaker, and shaken and cultured at 37°C, a $CO_2$ concentration of 5%, and 46 rpm.

After three days from the start of the shaking culture, 4 ml of a supernatant liquid medium was replaced with 4 ml of a new liquid medium. After five days from the start of the shaking culture, 4 ml of the supernatant liquid medium was removed from the culture plate. Then, 1.0 mL of TryPLE Express stripping solution was added to the culture plate and suspended by performing a pipetting operation. The suspension was added to a cell strainer together with the microcarrier to separate the microcarrier and the cell suspension. The number of cells contained in the obtained cell suspension was determined using a cell counter ("NucleoCounter NC-3000" manufactured by Chemometec).

<Criteria for cell culture evaluation>

AA: The number of cells is $1.6 \times 10^5$ or more
A: The number of cells is $1.2 \times 10^5$ or more and less than $1.6 \times 10^5$
B: The number of cells is $3.8 \times 10^4$ or more and less than $1.2 \times 10^5$
C: The number of cells is less than $3.8 \times 10^4$

(8) Breakage of microcarrier

The microcarrier after the evaluation in "(7) Evaluation of cell culture" was observed with a phase contrast microscope to confirm whether breakage occurred in the microcarrier. In the table, a case where the microcarrier in which breakage occurred was observed was described as "presence", and a case where the microcarrier in which breakage occurred was not observed was described as "absence". In Comparative Example 1, a broken microcarrier as shown in Fig. 2 was observed.
The details and the results are shown in the following Tables 1 and 2.

[Table 1]

| | | | Example 1 | Example 2 | Example 3 | Example 4 |
|---|---|---|---|---|---|---|
| Base particle | Type of base particle | | A | B | C | D |
| | Type of resin | | DVB | DVB | DVB | DVB |
| | Average particle diameter | μm | 100 | 200 | 300 | 400 |
| | Specific gravity | g/cm3 | 1.11 | 1.11 | 1.11 | 1.11 |
| Coating layer | Type of resin | | Resin X1 | Resin X1 | Resin X1 | Resin X1 |
| | Thickness | nm | 100 | 100 | 100 | 100 |
| Microcarrier | Specific gravity | $g/cm^3$ | 1.11 | 1.11 | 1.11 | 1.11 |
| | Average particle diameter | μm | 100 | 200 | 300 | 400 |
| | CV value of particle diameter | % | 1 | 1 | 1 | 1 |
| | Strength at break | mN | > 2000 | > 2000 | > 2000 | > 2000 |
| | Load at inflection point of compression displacement curve | mN | < | < | < | - |
| | Water absorption rate | wt% | 0.5 | 0.5 | 0.5 | 0.5 |
| Evaluation of cell culture (cell number) | | | A | A | AA | AA |
| Breakage of microcarrier | | | Absence | Absence | Absence | Absence |

[Table 2]

| | | | Example 5 | Example 6 | Example 7 | Comparative Example 1 | Comparative Example 2 |
|---|---|---|---|---|---|---|---|
| Base particle | Type of base particle | | E | C | F | G | C |
| | Type of resin | | PS97%/DVB3% | DVB | ACR | PS | DVB |
| | Average particle diameter | μm | 300 | 300 | 300 | 200 | 300 |
| | Specific gravity | g/cm$^3$ | 1.10 | 1.11 | 1.19 | 1.02 | 1.11 |
| Coating layer | Type of resin | | Resin X1 | Resin X2 | Resin X1 | Resin X1 | - |
| | Thickness | nm | 100 | 100 | 100 | 100 | - |
| Microcarrier | Specific gravity | g/cm3 | 1.10 | 1.11 | 1.19 | 1.02 | 1.11 |
| | Average particle diameter | μm | 300 | 300 | 300 | 200 | 300 |
| | CV value of particle diameter | % | 5 | 1 | 1 | 1 | 1 |
| | Strength at break | mN | 1500 | > 2000 | > 2000 | 800 | > 2000 |
| | Load at inflection point of compression displacement curve | mN | - | - | - | 500 | - |
| | Water absorption rate | wt% | 0.5 | 0.5 | 0.5 | 0.5 | 0.5 |
| Evaluation of cell culture (cell number) | | | AA | AA | AA | A | C |
| Breakage of microcarrier | | | Absence | Absence | Absence | Presence | Absence |

## EXPLANATION OF SYMBOLS

1: Microcarrier for cell culture
2: Base particle
3: Coating layer

## Claims

1. A microcarrier for cell culture comprising:

   a base particle; and
   a coating layer coating an outer surface of the base particle,
   the microcarrier for cell culture having a strength at break of 1000 mN or more.

2. The microcarrier for cell culture according to claim 1, wherein a compression displacement curve obtained when a compression test is performed has no inflection point at a load of 700 mN or less.

3. The microcarrier for cell culture according to claim 1 or 2, wherein a water absorption rate is 10% by weight or less.

4. The microcarrier for cell culture according to any one of claims 1 to 3, wherein the coating layer contains a synthetic resin.

5. The microcarrier for cell culture according to claim 4, wherein the synthetic resin has a polyvinyl alcohol derivative skeleton or a poly(meth)acrylic acid ester skeleton.

6. The microcarrier for cell culture according to any one of claims 1 to 5, wherein the coating layer contains a peptide moiety.

7. The microcarrier for cell culture according to any one of claims 1 to 6, wherein the base particle is a resin particle.

8. The microcarrier for cell culture according to any one of claims 1 to 7, wherein the base particle contains a polymer of a monomer having an ethylenically unsaturated group.

9. The microcarrier for cell culture according to claim 8, wherein the polymer of the monomer having the ethylenically unsaturated group is an acrylic resin, a divinylbenzene polymer, or a divinylbenzene copolymer.

10. The microcarrier for cell culture according to any one of claims 1 to 9, wherein a specific gravity is 1.0 g/cm$^3$ or more and 2.0 g/cm$^3$ or less.

11. The microcarrier for cell culture according to any one of claims 1 to 10, wherein an average particle diameter is 100 um or more and 1500 um or less.

12. The microcarrier for cell culture according to any one of claims 1 to 11, wherein a CV value of a particle diameter is 10% or less.

13. A cell culture method comprising adhering a cell to the microcarrier for cell culture according to any one of claims 1 to 12.

[FIG. 1.]

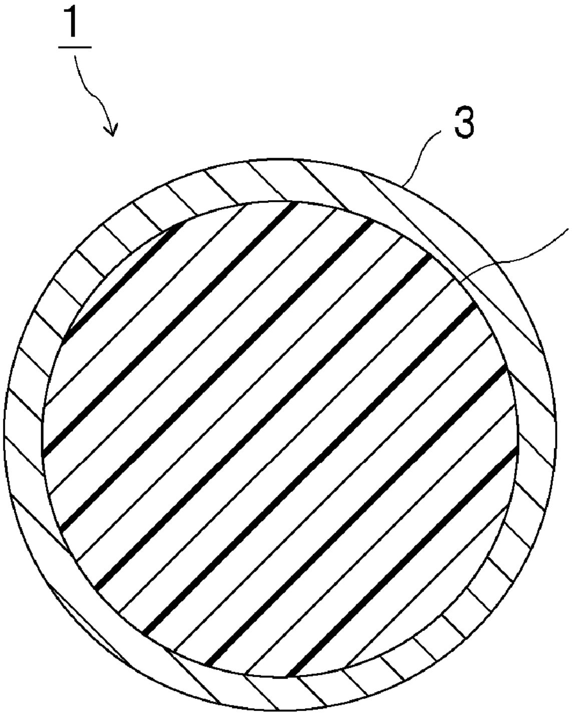


[FIG. 2.]

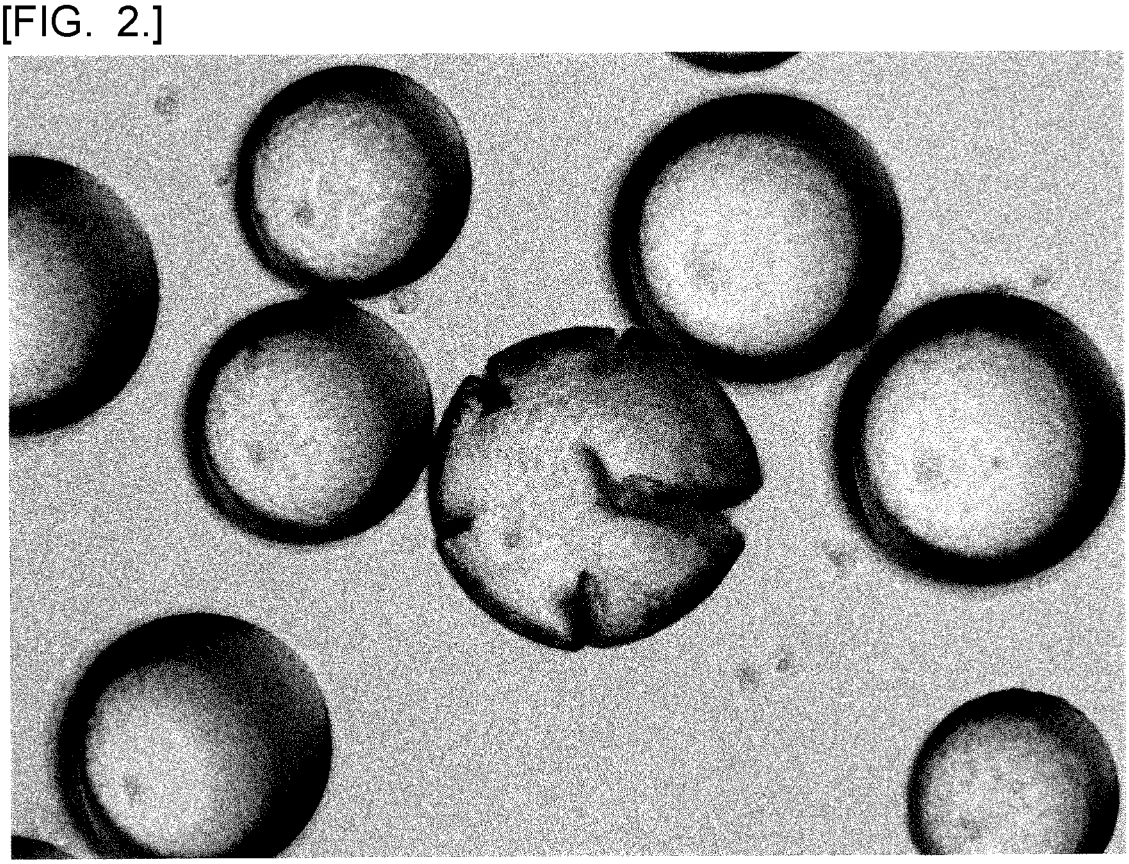

## INTERNATIONAL SEARCH REPORT

International application No.
**PCT/JP2022/004063**

**A. CLASSIFICATION OF SUBJECT MATTER**

***C12M 3/00***(2006.01)i; ***C07K 5/00***(2006.01)i; ***C07K 7/06***(2006.01)i; ***C12M 1/00***(2006.01)i; ***C12N 5/02***(2006.01)i; ***C12N 5/071***(2010.01)i
FI: C12M3/00 A; C07K5/00; C07K7/06; C12M1/00 C; C12N5/02 ZNA; C12N5/071

According to International Patent Classification (IPC) or to both national classification and IPC

**B. FIELDS SEARCHED**

Minimum documentation searched (classification system followed by classification symbols)

C12M3/00; C07K5/00; C07K7/06; C12M1/00; C12N5/02; C12N5/071

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII), Japio-GPG/FX, PubMed

**C. DOCUMENTS CONSIDERED TO BE RELEVANT**

| Category* | Citation of document, with indication, where appropriate, of the relevant passages | Relevant to claim No. |
|---|---|---|
| X | JP 2016-171794 A (THE NATIONAL INSTITUTE OF ADVANCED INDUSTRIAL SCIENCE AND TECHNOLOGY) 29 September 2016 (2016-09-29) claims 1-12, paragraphs [0012], [0013], [0045], [0109]-[0135] | 1-3, 6-13 |
| Y | claims 1-12, paragraphs [0012], [0013], [0045], [0109]-[0135] | 1-13 |
| Y | JP 03-043076 A (MITSUBISHI CHEMICAL CORP.) 25 February 1991 (1991-02-25) claims 1-2, examples 1-3, p. 520, left column, paragraph 2 to p. 521, left column, paragraph 2 | 1-13 |
| Y | WO 2020/230884 A1 (SEKISUI CHEMICAL CO., LTD.) 19 November 2020 (2020-11-19) claims 1-9, paragraphs [0093], [0153], examples 1-5 | 1-13 |
| A | WO 2020/203770 A1 (SEKISUI CHEMICAL CO., LTD.) 08 October 2020 (2020-10-08) entire text, all drawings | 1-13 |
| A | US 2012/0322145 A1 (THE REGENTS OF THE UNIVERSITY OF CALIFORNIA) 20 December 2012 (2012-12-20) entire text, all drawings | 1-13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2022** | **19 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/004063**

**C. DOCUMENTS CONSIDERED TO BE RELEVANT**

| Category* | Citation of document, with indication, where appropriate, of the relevant passages | Relevant to claim No. |
|---|---|---|
| A | ZHU, J., et al., Design and Synthesis of Biomimetic Hydrogel Scaffolds with Controlled Organization of Cyclic RGD Peptides., Bioconjugate Chemistry, 2009, vol. 20, pp. 333-339 entire text, all drawings | 1-13 |

Form PCT/ISA/210 (second sheet) (January 2015)

**INTERNATIONAL SEARCH REPORT**

International application No.
**PCT/JP2022/004063**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/004063**

| Patent document cited in search report | Publication date (day/month/year) | Patent family member(s) | Publication date (day/month/year) |
|---|---|---|---|
| JP 2016-171794 A | 29 September 2016 | (Family: none) | |
| JP 03-043076 A | 25 February 1991 | (Family: none) | |
| WO 2020/230884 A1 | 19 November 2020 | WO 2020/230886 A1<br>claims 1-16, paragraph [0141], examples 1-17<br>WO 2020/230885 A1 | |
| WO 2020/203770 A1 | 08 October 2020 | (Family: none) | |
| US 2012/0322145 A1 | 20 December 2012 | WO 2010/148346 A2<br>entire text, all drawings | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011017167 A1
- WO 2011017050 A1


### Non-patent literature cited in the description


- *The Journal of Cell Biology,* September 1995, vol. 130 (5), 1189-1196
- *Protein, Nucleic Acid and Enzyme,* 2000, vol. 45 (15), 2477
- *Medicina Philosophica,* 1990, vol. 9 (7), 527-535
- *Journal of Osaka Women's and Children's Hospital,* 1992, vol. 8 (1), 58-66